(19) Europäisches Patentamt
European Patent Office
Office européen des brevets

(11) Numéro de publication: **0 179 697 B1**

# (12) FASCICULE DE BREVET EUROPEEN

(45) Date de publication du fascicule du brevet: **22.03.89**

(21) Numéro de dépôt: **85401886.8**

(22) Date de dépôt: **26.09.85**

(51) Int. Cl.⁴: **C 07 D 275/02**, C 07 D 275/04, C 07 D 513/04, A 61 K 31/425, A 61 K 31/435, A 61 K 7/06, A 61 K 7/48 // (C07D513/04, 275:00, 221:00)

## (54) Nouveaux dérivés de l'acide rétinoique, leur procédé de préparation et compositions médicamenteuse et cosmétique les contenant.

(30) Priorité: **26.09.84 LU 85556**

(43) Date de publication de la demande: **30.04.86 Bulletin 86/18**

(45) Mention de la délivrance du brevet: **22.03.89 Bulletin 89/12**

(84) Etats contractants désignés: **BE CH DE FR GB IT LI NL SE**

(56) Documents cités:
**DE-A- 3 342 538**
**GB-A- 2 087 388**
**GB-A- 2 118 554**
**US-A- 4 105 431**

**EXPERIENTIA, vol. 34, no. 9, 15 septembre 1978, pages 1105-1119, Bâle, CH; H. MAYET et al.: "Retinoids a new class of compounds with prophylactic and therapeutic activities in oncology and dermatology"**
**Cancer Research 40 (1980), 3413-3425**
**J. Med. Chem. 20 (1977) No. 7, 918-925**

**Le dossier contient des informations techniques présentées postérieurement au dépôt de la demande et ne figurant pas dans le présent fascicule.**

(73) Titulaire: **CENTRE INTERNATIONAL DE RECHERCHES DERMATOLOGIQUES C.I.R.D. Groupement d'Intérêt Economique dit:, Sophia Antipolis, F-06560 Valbonne (FR)**

(72) Inventeur: **Shroot, Braham Villa 35, Hameaux du Val Bosquet Chemin du Val Bosquet, F-06600 Antibes (FR)**
Inventeur: **Lang, Gérard, 44, avenue Lacour, F-95210 Saint-Gratien (FR)**
Inventeur: **Maignan, Jean, 8, rue Halévy, F-93290 Tremblay-lès-Gonesse (FR)**
Inventeur: **Colin, Michel, 10, Allée Cécile, F-93190 Livry-Gargan (FR)**

(74) Mandataire: **Peuscet, Jacques et al, Cabinet Peuscet 68, rue d'Hauteville, F-75010 Paris (FR)**

EP 0 179 697 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux composés chimiques qui sont des amides ou des esters de l'acide rétinoïque. Lorsqu'il s'agit d'amides, l'atome d'azote de la fonction amide est incorporé dans un hétérocycle isothiazolone; lorsqu'il s'agit d'esters, les nouveaux composés sont des rétinoates d'(hydroxy-2' alkyl)-2 isothiazolone. L'invention concerne également un procédé de préparation permettant d'obtenir ces nouveaux composés. L'invention concerne, enfin, ces nouveaux composés pour l'utilisation soit en cosmétique, soit comme préparations pharmaceutiques dans le traitement des affections dermatologiques liées à un désordre de la kératinisation (différenciation-prolifération) et dans le traitement des affections dermatologiques ou autres à composante inflammatoire et/ou immuno-allergique, ainsi que comme préparations pharmaceutiques pour le domaine ophtalmologique, notamment dans le traitement des cornéopathies.

L'action thérapeutique de la vitamine A sous sa forme acide (acide rétinoïque), aldéhyde ou alcool, est bien connue en dermatologie (voir à cet égard la publication «EXPERIENTIA», volume 34, pages 1105–1119 (1978) ); cette action dans le traitement des proliférations cutanées, de l'acné, du psoriasis et des affections analogues sera désignée ci-après par l'expression générique «action de type rétinoïde». Certains esters de l'acide rétinoïque sont connus; voir Cancer Research 40 (1980), 3413–3425 et J. Med. Chem. 20 (1977) N° 7, p. 918–925. On a constaté que des produits ayant une structure analogue à la vitamine A présentaient également une action de type rétinoïde.

La formule de l'acide rétinoïque:

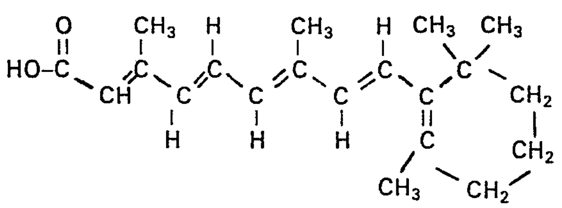

sera représentée dans la présente description et dans les revendications sous la forme condensée correspondant à la formule (II)

$$Ret{-}CO_2H \qquad II$$

où l'on a simplement explicité la fonction acide, le reste de la molécule de l'acide rétinoïque étant représenté par «Ret».

On sait que les isothiazolones de formule (III)

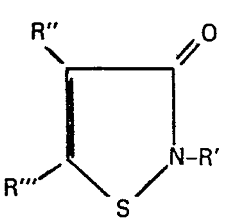

III

formule dans laquelle R', R'' et R''' représentent divers substituants, sont des hétérocycles connus, qui ont de bonnes propriétés bactéricides; par ailleurs, on sait qu'un certain nombre de ces produits preésentent une bonne activité sur les germes de l'acné.

Selon l'invention, on a constaté que si l'on associait chimiquement les produits de formules (II) et (III) ci-dessus indiquées, on obtenait un composé nouveau qui possédait à la fois les propriétés des composés de formule (II) et des composés de formule (III). Mais, en outre, on a constaté que les nouveaux composés ainsi obtenus avaient la propriété inattendue de réduire, voire de supprimer, l'irritation due à l'utilisation de l'acide rétinoïque, notamment en application topique; cette propriété surprenante a été démontrée par des tests sur les lapins. La société demanderesse a émis l'hypothèse que les nouveaux produits selon l'invention pouvaient, dans l'organisme traité, se couper en redonant naissance aux composés de formules (II) et (III), mais que cette coupure s'effectuant in situ de façon lente, permet un étalement de l'action de chacun des composés de formules (II) et (III) et, par conséquent, une réduction de l'irritation causée par l'action de l'acide rétinoïque. Cette hypothèse est ici donnée sans qu'elle puisse en aucun cas être considérée comme une limitation de l'invention. Il s'agit simplement d'une explication possible de la caractéristique surprenante des nouveaux composés de l'invention.

La présente invention a, donc, pour objet le produit industriel nouveau que constitue un composé chimique correspondant à la formule générale (IV)

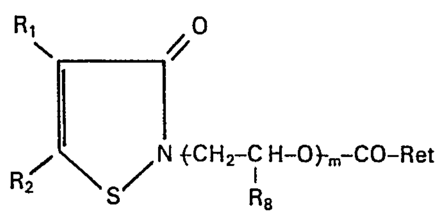

IV

formule dans laquelle $R_1$ et $R_2$ sont identiques ou différents et représentent:

A) pris isolément: un atome d'hydrogène, un radical alkyle linéaire ou ramifié $C_1$–$C_4$, un radical phényle non substitué, ou un radical phényle substitué par un atome d'halogène, par un radical alkyle $C_1$–$C_4$ ou par un radical alkoxy $C_1$–$C_4$;

B) pris ensemble:

a) ou bien un enchaînement polyméthylène-$(CH_2)_n$-, n étant égal à 3 ou 4;

b) ou bien un enchaînement –CH=CH–CH=CH–, le cycle benzénique ainsi formé pouvant être substitué par des radicaux $R_3$ et $R_4$, identiques ou différents, les radicaux $R_3$ et $R_4$ représentant un atome d'hydrogène, un atome d'halogène, un radical alkyl $C_1$–$C_4$, un radical alkoxy $C_1$–$C_4$, un radical nitro ou un radical carboxylique;

c) ou bien un enchaînement de formule (V):

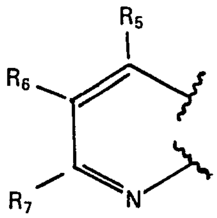

V

formule dans laquelle $R_5$, $R_6$ et $R_7$ sont identiques ou différents et représentent un atome d'hydrogène ou un radical alkyle linéaire ou ramifié $C_1$–$C_4$;

m pouvant avoir les valeurs 0 ou 1;

$R_8$ correspondant à un atome d'hydrogène, un radical méthyle, un radical hydroxyméthyle ou un groupement-$CH_2O$–CO-Ret,

Ret représentant le reste de la molécule d'acide rétinoïque dont on a enlevé la fonction acide.

Dans le cas où $R_1$ et $R_2$ forment ensemble un enchaînement polyméthylène, les composés de formule (IV) sont des dérivés de triméthylène-4,5 isothiazolone ou de tétraméthylène-4,5 isothiazolone.

Dans le cas où $R_1$ et $R_2$ pris ensemble forment un cycle benzénique et m=1, les composés de formule (IV) sont des dérivés de benzoisothiazolone de formule (VII)

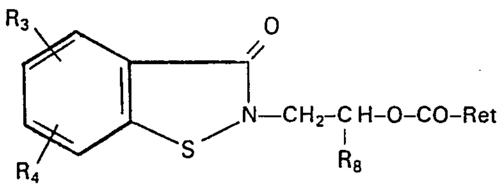

VII

$R_3$, $R_4$ et $R_8$ ayant les significations précédemment indiquées.

Dans le cas où $R_1$ et $R_2$ pris ensemble forment un enchaînement de formule (V), les composés de formule (IV) sont des dérivés d'isothizolopyridinone correspondant à la formule (VIII)

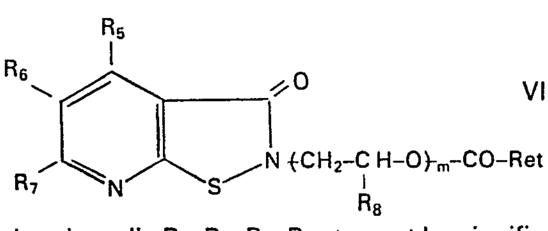

VIII

dans laquelle $R_5$, $R_6$, $R_7$, $R_8$ et m ont les significations précédemment indiquées.

Lorsque m=0, les composés de formule (IV) sont des N-rétinoyl-isothiazolones, donc des amides de l'acide rétinoïque.

Lorsque m=1, les composés de formule (IV) sont des rétinoates, donc des esters de l'acide rétinoïque, répondant à la formule (IX)

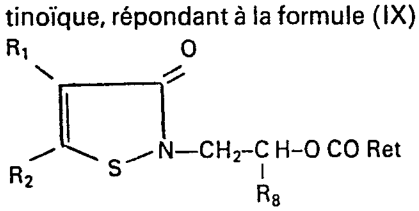

IX

dans laquelle $R_1$, $R_2$ et $R_8$ ont des significations précédemment indiquées.

Parmi les composés de formule (IV), on préfère les composés suivants:

1) les composés pour lesquels $R_1$ représente un atome d'hydrogène et $R_2$ représente H $CH_3$ ou $C_6H_5$;

2) le composé pour lequel $R_1$ et $R_2$ forment une chaîne polyméthylène $-(CH_2)_3-$;

3) les composés dans lesquels $R_1$ et $R_2$ forment ensemble un cycle aromatique et qui sont des dérivés de la benzoisothiazolone ou de l'isothiazolopyridinone.

Les composés de formule (IV) sont obtenus par réaction d'une isothiazolone de formule (X) ou (XI)

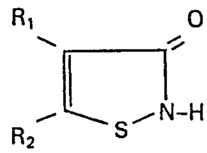

X

ou

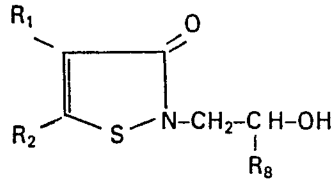

XI

sur le chlorure d'acide de l'acide rétinoïque.

Les réactions sur les composés des formules (X) ou (XI) sont effectuées soit à la température ordinaire en présence d'environ 1 équivalent basique dans un solvant aromatique, tel que le toluène, ou dans un éther, tel que le tétrahydrofuranne, soit en portant une solution toluénique sensiblement équimoléculaire de chlorure de rétinoyle et d'isothiazolone à une température comprise entre 70 °C et 80 °C.

Lorsque l'on opère à température ordinaire, on peut utiliser comme base la pyridine ou la triéthylamine et on ajoute alors environ 1 équivalent de chlorure de rétinoyle au mélange équimoléculaire isothiazolone-amine tertiaire. On peut également utiliser comme base l'hydrure de sodium. Dans ce cas, on préfère former au préalable le sel de sodium de l'isothiazolone en traitant celle-ci, solubilisée dans le tétrahydrofuranne, par environ 1 équivalent d'hydrure de sodium; lorsque le dégagement del'hydrogène a cessé, on considère que toute l'isothiazolone est transformée en son sel de sodium; on ajoute alors à celui-ci environ 1 équivalent de chlorure de rétinoyle.

Ces réactions sont conduites à l'abri de l'humidité de l'air et de la lumière. Leur évolution est suivie par chromatographie sur couche mince (gel de silice «F254»). Les produits obtenus sont isolés selon des procédés classiques. Le mélange réactionnel est lavé à l'eau; la phase organique est séchée, filtrée, concentrée et déposée sur une colonne de chromatographie de gel de silice; suivant la polarité du produit attendu, celui-ci peut être

élué au toluène ou au chlorure de méthylène. Après concentration des phases d'élution, le solide isolé peut être recristallisé dans un mélange toluène-hexane.

Les isothiazolones de formule (X) sont des produits connus et sont préparés comme indiqué dans l'état de la technique, à savoir:

a) pour certaines isothiazolones, article Sheldon N. Lewis et col., J. Heter. Chem., 1971 p. 571;

b) pour les benzoisothiazolones, article de R. Fischer et H. Hurni, Arzneimittel Forschung, 1964, 14, p. 1301 et article M. Clelland E.W., J. Chem. Soc. 1926, p. 923;

c) pour les isothiazolopyridinones, brevet des Etats Unis d'Amérique n° 3 965 107;

d) pour les polyméthylène-isothiazolones, brevet français 2 492 376.

Les isothiazolones de formuel (XI) comportant une chaîne hydroxyalkyle ou dihydroxyalkyle en position 2, peuvent être préparées à partir des isothiazolones primaires que l'on fait réagir sur un époxyde tel que l'oxyde d'éthylène, l'oxyde de propylène ou le glycidol.

Le chlorure de rétinoyle utilisé dans le procédé de fabrication selon l'invention est préparé selon un procédé classique en faisant réagir le trichlorure de phosphore sur l'acide rétinoïque. Les deux isomères 13-cis et tout-trans sont des produits commerciaux.

Selon l'invention, on a constaté que les composés de formule (IV) ont une action de type rétinoïde, mais ne présentent que très peu de caractéristiques irritantes; ils conviennent donc particulièrement bien pour traiter les affections dermatologiques liées à un désordre de la kératinisation (différenciation-prolifération); on a également constaté que les produits de formule (IV) permettaient de traiter les affections dermatologiques, ou autres, à composante inflammatoire et/ou immuno-allergique. Les composés selon l'invention sont, notamment, utiles pour traiter les acnés vulgaires, comédoniennes ou polymorphes, les acnés séniles, solaires, et les acnés médicamenteuses ou professionnelles, les formes étendues et/ou sévères de psoriasis, et les autres troubles de la kératinisation, et notamment les ichtyoses et états ichtyosiformes, la maladie de Darier, les kératodermies palmo-plantaires, les leucoplasies et états leucoplasiformes, le lichen plan, toutes proliférations dermatologiques bénignes ou malignes, sévères ou étendues, ils sont également actifs dans le traitement du psoriasis rhumatoïde. Enfin, ces produits trouvent une application dans le domaine ophtalmoligique, notamment pour le traitement des cornéopathies. L'invention porte donc également sur des compositions médicamenteuses contenant les composés de formule (IV).

La présente invention a donc aussi pour objet une nouvelle composition médicamenteuse, destinée notamment au traitement des affections susmentionnées, caractérisée par le fait qu'elle comporte, dans un support pharmaceutiquement acceptable, au moins un composé de formule (IV) et/ou un de ses sels.

Lorsque les composés selon l'invention sont utilisés par voie topique, on observe une bonne activité des composés selon l'invention sur une très grande gamme de dilution; on peut utiliser, notamment, des concentrations en composé(s) actif(s) allant de 0,0005% à 2% en poids. Il est bien entendu possible d'utiliser des concentrations supérieures lorsque ceci est rendu nécessaire pour une application thérapeutique particulière; toutefois, les concentrations préférées en principe actif sont comprises entre 0,002% et 1% en poids. Ces compositions utilisables par voie topique sont présentées avantageusement, sous la forme d'onguents, de gels, de crèmes, de pommades, de poudres, de teintures, de solutions, de suspensions, d'émulsions, de lotions, de sprays, de timbres, de tampons imbibés. Les composés selon l'invention sont mélangés à des supports inertes non toxiques, généralement liquides ou pâteux, appropriés au traitement par voie topique.

Les composés selon l'invention peuvent être utilisés par voie entérale. Par voie orale, on administre les composés selon l'invention à raison d'environ 2 µg jusqu'à 2 mg par jour et par kg de poids corporel; une posologie excessive peut se manifester sous la forme d'une hypervitaminose A reconnaissable à ses symptômes et pouvant faire craindre une toxicité hépatique nécessitant une contrôle biologique de la fonction hépatique. La dose nécessaire peut être administrée en une ou plusieurs prises. Pour l'administration par voie orale, les formes appropriées sont, par exemple, les comprimés, les gélules, les dragées, les sirops, les suspensions, les émulsions, les solutions, les poudres, les granulés; un mode d'administration préféré consiste à utiliser des gélules contenant de 0,1 mg à environ 1 mg de substance active.

Les composés selon l'invention peuvent également être administrés par voie parentérale sous forme de solutions ou suspensions pour perfusions ou injections intraveineuses ou intramusculaires. Dans ce cas, on administre les composés selon l'invention à raison d'environ 2 µg jusqu'à 2 mg par jour et par kg de poids corporel; un mode d'administration préféré consiste à utiliser des solutions ou suspensions contenant de 0,01 mg à 1 mg environ de substance active par ml.

Lorsque les composés selon l'invention sont utilisés par voie oculaire, ils se présentent avantageusement sous forme de solutions ou de poudres à diluer pour collyres.

Le support pharmaceutiquement acceptable peut comporter de l'eau, de la gélatine, du lactose, de l'amidon, du talc, de la vaseline, de la gomme arabique, des polyalcoylèneglycols, du stéarate de magnésium. Les comprimés, poudres, dragées, granulés ou gélules peuvent contenir des liants, des charges, des supports pulvérulents. Les solutions, crèmes, suspensions, émulsions ou sirops peuvent contenir des diluants, des solvants, des épaississants.

Les composés de formule (IV) selon l'invention

trouvent également une application dans le domaine cosmétique, en particulier dans l'hygiène corporelle et capillaire et, notamment, dans le traitement de l'acné, des peaux physiologiquement sèches, des séborrhées, de la chute des cheveux ou pour la repousse des cheveux. Enfin, ils ont un pouvoir préventif et curatif contre les effets néfastes du soleil.

La présente invention a donc aussi pour objet une nouvelle composition cosmétique, caractérisée par le fait qu'elle comporte, dans un support cosmétiquement acceptable, au moins un composé de formule (IV) et/ou un de ses sels; cette composition peut se présenter sous forme de lotion, gel, crème, savon, shampooing ou analogue.

La concentration en composé de formule (IV) dans ces compositions cosmétiques est comprise entre 0,0005 et 2% en poids et, de préférence, entre 0,01 et 1% en poids par rapport au poids total de la composition.

Dans le traitement des désordres susmentionnés, les composés selon l'invention, utilisés dans les compositions ci-dessus définies, agissent en accroissant la production épithéliale folliculaire des cellules non-adhérentes, délogeant ainsi et faisant partir le contenu du comédon acnéique. Ces composés réduisent la taille des glandes sébacées et inhibent partiellement la sécrétion du sébum.

Les compositions selon l'invention peuvent contenir des additifs inertes ou même pharmacodynamiquement ou cosmétiquement actifs, et notamment:

– des agents hydratants comme la thiamorpholinone et ses dérivés ou l'urée.

– des agents antiséborrhéïques ou antiacnéïques tels que ceux décrits dans les brevets français 1 472 021, 1 505 874, 1 560 250, 2 002 461, 2 035 799, 2 011 940, 2 060 407, 2 126 996, 2 133 991, 2 133 992, 2 139 876, 2 158 018, 2 296 406, 2 428 436, 2 468 362, 2 446 277, 2 447 189, et l'USP 2 332 418 et en particulier, la S-carboxyméthylcystéïne, la S-benzylcystéamine, leurs sels et leurs dérivés, la tioxolone, ou encore le peroxyde de benzoyle.

– des antibiotiques tels que l'érythromycine et ses esters, par exemple ceux décrits dans le brevet des Etats Unis d'Amérique 2 862 921 ou la demande de brevet français 85 05785, la néomycine, les tétracyclines ou les polyméthylène-4,5 isothiazolinones-3 telles que décrites dans le brevet français 2 492 376.

– des agents favorisant la repousse des cheveux, comme le «Minoxidil» (diamino-2,4, pipéridino-6 pyrimidine oxyde-3) et ses dérivés, le Diazoxide (chlorméthyl-3 benzothiadiazine-1,2,4 dioxyde-1,1, le Phénytoïn (diphényl-5,5 imidazolidinedione-2,4), l'iodure d'oxapropanium ou encore l'anthraline et ses dérivés.

– des agents anti-inflammatoires (stéroïdiens et non-stéroïdiens).

– des caroténoïdes et, notamment, le β-carotène.

– des agents anti-psoriasiques comme les acides eicosaté traynoïque -5,8,11,14 et triynoïque-5,8,11, leurs esters et leurs amides, l'anthraline et ses dérivés tels que ceux décrits dans les brevets français 2 113 952, 2 492 372, 2 492 373, 2 495 934, 2 499 556, ou les demandes de brevet français 84.09203 et 84.10324 ou le brevet des Etats Unis d'Amérique 4 299 846, les dérivés naphtaléniques et naphtoquinoniques tels que ceux décrits dans le brevet des Etats Unis d'Amérique 4 229 478, le brevet européen 7985 ou dans le J.I.D. 84(4) 358 (1985).

Les compositions selon l'invention peuvent également contenir des agents de sapidité, des agents conservateurs, des agents stabilisants, des agents régulateurs d'humidité, des agents régulateurs de pH, des agents modificateurs de pression osmotique, des agents émulsionnants, des filtres UV-A et UV-B tels que ceux décrits dans les brevets français 1 179 387 ou 2 528 420 et des agents anti-oxydants tels que l'α-tocophérol, le butylhydroxyanisole ou le butylhydroxytoluène.

Pour mieux faire comprendre l'objet de l'invention, on va en décrire maintenant plusieurs exemples de réalisation. L'exemple A fournit la préparation du chlorure de rétinoyle et ne fait pas partie de l'invention.

Exemple A

Préparation du chlorure d'acide de l'acide tout-trans rétinoïque

A une solution de 3 g d'acide tout-trans rétinoïque dans 30 $cm^3$ de toluène anhydre, agitée à l'abri de la lumière et de l'humidité de l'air, on ajoute, goutte à goutte, 0,3 $cm^3$ de trichlorure de phosphore. A la fin de l'introduction, le mélange est alors porté pendant une demi-heure à 60 °C, temps au bout duquel tout l'acide est transformé.

Cette solution toluénique est alors transvasée directement dans une ampoule à brome pour être introduite sur une solution contenant l'isothiazolinone.

Exemple 1

Préparation de la (tout-trans rétinoyl)-2 benzoisothiazoline-4 one-3

Dans un tricol muni d'une arrivée d'azote, d'une agitation magnétique, d'une ampoule à brome, on prépare, à l'abri de la lumière, une solution de 2,26 g de benzoisothiazolone (0,015 mole) et de 1,2 $cm^3$ de pyridine dans 75 $cm^3$ de toluène anhydre. A ce mélange agité, on ajoute alors, goutte à goutte, une solution brute de 0,015 mole de chlorure de rétinoyle (tout-trans).

On constate une légère exothermicité et le chlorhydrate de pyridinium précipite au fur et à mesure de sa formation. Le mélange est encore agité pendant trois heures à température ordinaire.

On vérifie que le produit de départ est totalement transformé par chromatographie couche mince. Le mélange réactionnel est lavé à l'eau jusqu'à pH neutre. La phase organique est alors décantée, séchée sur sulfate de magnésium, puis concentrée et enfin déposée sur une colonne de chromatographie de gel de silice. Le produit attendu est élué au toluène.

Le solide isolé après évaporation des fractions d'élution est cristallisé dans le mélange toluène-hexane.

On obtient 1,5 g de cristaux jaune orange fondant à 172 °C.

Les spectres de résonance magnétique nucléaire $^{13}C$ et $^{1}H$ correspondent à une structure entièrement trans de la chaîne rétinoyle. Analyse élémentaire:

| | C | H | N | O | S |
|---|---|---|---|---|---|
| Calculé pour $C_{27}H_{31}NO_2S$ | 74,79 | 7,21 | 3,23 | 7,38 | 7,39 |
| Trouvé | 74,74 | 7,27 | 3,31 | 7,50 | 7,27 |

Exemple 2

Préparation de la (tout-trans rétinoyl)-2 triméthylène-4,5 isothiazoline-4 one-3

On traite 0,01 mole (1,41 g) de triméthylène-4,5 isothiazoline-4 one-3 par un équivalent de chlorure de rétinoyle en présence de pyridine dans les mêmes conditions que celles décrites précédemment pour la benzoisothiazolone.

Le produit attendu est purifié par passage sur colonne de gel de silice. Il est élué au chlorure de méthylène et recristallisé dans un mélange toluène-hexane On obtient 1,5 g de cristaux jaunes fondant à 138 °C, dont les spectres de résonance magnétique nucléaire $^{13}C$ et $^{1}H$ correspondent à une structure entièrement trans de la chaîne rétinoyle.

L'analyse élémentaire suivante correspond à un produit hydraté:

| | C | H | N | O | S |
|---|---|---|---|---|---|
| Calculé pour $C_{26}H_{33}NO_2S$, $^1/_4$ $H_2O$ | 72,94 | 7,89 | 3,27 | 8,41 | 7,49 |
| Trouvé | 72,86 | 7,91 | 3,45 | 8,73 | 7,09 |

Exemple 3

Préparation de la («tout-trans» rétinoyl)-2 triméthylène-4,5 isothiazoline-4 one-3

Dans un tricol muni d'une arrivée d'azote, d'une agitation magnétique, d'une ampoule à brome, on place (0,035 mole) et 350 $cm^3$ de tétrahydrofuranne anhydre. A cette solution préparée, on ajoute alors 1,2 équivalent molaire d'hydrure de sodium. Lorsque la formation du sel de sodium de la triméthylène-4,5 isothiazolinone-3 est terminée (fin de dégagement d'hydrogène), on ajoute goutte à goutte, à l'aide de l'ampoule, à l'abri de la lumière, la solution contenant le chlorure de rétinoyle (tout-trans: 1,2 équivalent). Deux heures après la fin de l'introduction du chlorure d'acide, on vérifie par chromatographie sur couche mince que la réaction est terminée. On ajoute alors 3 $cm^3$ d'acide acétique pour détruire le reste éventuel d'hydrure de sodium et le mélange réactionnel est traité comme dans les exemples précédents.

Après chromatographie sur colonne de gel de silice et cristallisation, on obtient 3 g de («tout-trans» rétinoyl)-2 triméthylène-4,5 isothiazoline-4 one-3 fondant également à 138 °C.

Exemple 4

Préparation de la («tout-trans» rétinoyl)-2 isothiazolo-(5,4 b) pyridinone-3

On opère comme dans le cas de la triméthylène-4,5 isothiazolone en préparant, dans un premier temps, le sel de sodium à partir de 3 g d'isothiazolo-(5,4 b) pyridinone-3 que l'on traite par 1,2 équivalent d'hydrure de sodium dans 250 $cm^3$ de tétrahydrofuranne. Lorsque le dégagement d'hydrogène a cessé, on ajoute alors lentement 1,2 équivalent de chlorure de rétinoyle tout-trans préparé séparément. L'agitation est maintenue pendant trois heures et le milieu réactionnel est abandonné pendant une nuit. Le lendemain, il est traité comme dans les exemples précédents et la (tout-trans rétinoyl)-2 isothiazolo-(5,4 b) pyridinone-3 est purifiée par chromatographie sur colonne de gel de silice, suivie d'une cristallisation dans le mélange toluène-hexane.

On obtient 1 g de paillettes nacrées oranges dont le point de fusion est de 198 °C.

Les spectres de masse et de résonance magnétique nucléaire correspondent à la structure attendue.

Exemple 5

Préparation de la («tout-trans» rétinoyl)-2 tétraméthylène-4,5 isothiazoline-4 one-3

A une solution de 1 g (0,006 mole) de tétraméthylène-4,5 isothiazoline-4 one-3 dans 40 $cm^3$ de toluène anhydre agitée à l'abri de la lumière et de l'humidité de l'air, on ajoute un équivalent de chlorure de rétinoyle solubilisé dans 20 $cm^3$ de toluène.

Le mélange réactionnel est alors porté pendant deux heures à une température de 80 °C. La majorité du produit de départ est transformée. Le toluène est ensuite évaporé sous pression réduite. Le produit obtenu est solubilisé par du chlorure de méthylène et la solution est déposée sur une colonne de gel de silice. La «tout-trans» rétinoyl-2 tétraméthylène-4,5 isothiazolone est éluée au chlorure de méthylène. Aprés concentration des phases d'élution, le produit est recristallisé dans un mélange toluène-hexane. On obtient 500 mg de cristaux jaune orange fondant à 108 °C.

Le spectre de masse (m/e: 437) et le spectre de résonance magnétique nucléaire $^{1}H$ 250 MHz correspondent à la structure attendue.

Exemple 6

Préparation de la («toute-trans» rétinoyl)-2 isothiazolone-4 one-3

Ce produit est préparé de la même façon que le dérivé décrit à l'exemple 4. Un mélange équimoléculaire de 1 g d'isothiazoline-4 one-3 (0,01 mole) et de chlorure de rétinoyle dans 60 $cm^3$ de toluène anhydre est porté à 80 °C pendant deux heures. Ensuite, le produit est purifié de la même façon que dans l'exemple précédent. On obtient 350 mg de cristaux jaune orange fondant à 98 °C.

Le spectre de masse (m/e: 383) et le spectre de résonance magnétique nucléaire $^1H$ 250 MHz correspondent à la structure attendue.

Exemple 7

Préparation de la (tout-trans rétinoyloxy-2' éthyl)-2 benzoisothiazolone

A une solution de 1,95 g d'(hydroxy-2' éthyl)-2 benzoisothiazolone (0,01 mole) dans 100 $cm^3$ de toluène anhydre agitée à l'abri de la lumière et de l'humidité de l'air en présence de 3,6 $cm^3$ de pyridine, on ajoute un équivalent de chlorure de l'acide «tout-trans» rétinoïque solubilisé dans 50 $cm^3$ de toluène. Le mélange est agité pendant une heure à la température ordinaire et, ensuite, pendant sept heures à une température comprise entre 60 et 70 °C. La phase organique est lavée, puis séchée sur sulfate de magnésium. Elle est ensuite concentrée sous pression réduite et déposée sur une colonne de gel de silice. Le produit attendu est élué au chlorure de méthylène.

Après concentration, on obtient 1,5 g de rétinoate «tout-trans» sous forme d'un produit jaune très visqueux. Analyse élémentaire:

| | C | H | N | O | S |
|---|---|---|---|---|---|
| Calculé pour $C_{29}H_{35}NO_3S$ | 72,92 | 7,38 | 2,93 | 10,04 | 6,71 |
| Trouvé | 72,72 | 7,42 | 2,97 | 10,11 | 6,71 |

Exemple 8

On prépare une lotion anti-séborrhéique de la façon suivante:

A une solution constituée de 10 $cm^3$ d'éthanol à 95° et de 30 $cm^3$ de polyéthylèneglycol (masse moléculaire: environ 400) contenant 20 mg de butylhydroxytoluène, on ajoute 0,1 g du composé de l'exemple 4.

Après solubilisation sous agitation, on applique la lotion sur l'ensemble de la chevelure.

De préférence, on effectue un traitement deux fois par jour. Après 15 jours de traitement, on constate un résultat satisfaisant.

Cette composition peut être appliquée également sur peaux grasses.

Exemple 9

On prépare la composition suivante:

- Composé de l'exemple 2 — 0,1 g
- Hydroxypropyl cellulose vendue par la Société HERCULES sous le nom de «KLUCEL HF» — 2,00 g
- Eau/éthanol (50/50) qsp — 100 g

On obtient ainsi un gel utilisable pour le traitement de l'acné et applicable une à trois fois par jour; on obtient de bons résultats dans un délai compris entre 6 et 12 semaines selon la gravité du cas traité.

Exemple 10

On prépare la composition suivante:

- Composé de l'exemple 7 — 0,5 g
- Mélange d'alcools de lanoline émulsifs et de cires et d'huiles raffinées à base d'hydrocarbures vendu par la société B.D.F. MEDICAL sous le nom de «EUCERIN anhydre» — 40 g
- Conservateurs qs
- Eau déminéralisée stérile qsp — 100 g

On obtient ainsi une suspension non-ionique constituant une crème. Cette crème, utilisée pour le traitement du psoriasis, donne de bons résultats dans un délai de 30 jours par applications une à trois par jour.

Exemple 11

On prépare la composition suivante:

- Composé de l'exemple 1 — 0,02 g
- Polyéthylène glycol (poids moléculaire = 400) — 60 g
- Polyéthylèneglycol (poids moléculaire = 4000) — 25 g
- Huile de vaseline qsp — 100 g

On obtient ainsi un onguent éliminable à l'eau. Cette préparation, utilisée sur les peaux ichtyosiques, donne de bons résultats.

Exemple 12

On prépare la composition suivante:

- Composé de l'exemple 7 — 0,001 g
- Amidon de maïs — 0,150 g
- Stéarate de magnésium — 0,250 g
- Saccharose qsp — 0,500 g

On obtient une poudre conditionnée dans une gélule composée de gélatine et de dioxyde de titane.

On administre à un individu adulte une à trois gélules par jour pour le traitement du psoriasis et on constate une amélioration significative au bout de 30 jours environ.

**Revendications**

1. Composé chimique correspondant à la formule générale (IV)

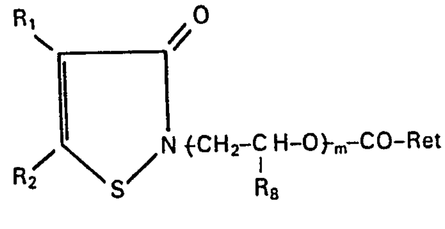

IV

formule dans laquelle $R_1$ et $R_2$ sont identiques ou différents et représentent:

A) pris isolément: un atome d'hydrogène, un radical alkyle $C_1$–$C_4$ linéaire ou ramifié, un radical

phényle non substitué, ou un radical phényle substitué parun atome d'halogène, par un radical alkyle $C_1$–$C_4$ ou par un radical alkoxy $C_1$–$C_4$;

B) pris ensemble:

a) ou bien un enchaînement polyméthylène-$(CH_2)_n$-, n étant égal à 3 ou 4;

b) ou bien un enchaînement –CH=CH–CH=CH–, le cycle benzénique ainsi formé pouvant être substitué par des radicaux $R_3$ et $R_4$ représentant un atome d'hydrogène, un atome d'halogène, un radical alkyle $C_1$–$C_4$, un radical alkoxy $C_1$–$C_4$, un radical nitro ou un radical carboxylique:

c) ou bien un enchaînement de formule (V)

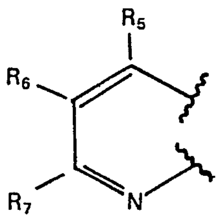

V

formule dans laquelle $R_5$, $R_6$ et $R_7$ sont identiques ou différents et représentent un atome d'hydrogène ou un radical alkyle $C_1$–$C_4$ linéaire ou ramifié;

m pouvant avoir les valeurs 0 ou 1;

$R_8$ correspondant à un atome d'hydrogène, un radical méthyle, un radical hydroxyméthyle ou un groupement $-CH_2O-CO-$Ret;

– Ret représentant le reste de la molécule d'acide rétinoïque dont on a enlevé la fonction acide.

2. Composé selon la revendication 1, caractérisé par le fait que $R_1$ représente un atome d'hydrogène et $R_2$ est choisi dans le groupe formé par un atome d'hydrogène et un radical $CH_3$ ou $C_6H_5$.

3. Composé selon la revendication 1, caractérisé par le fait que $R_1$ et $R_2$ forment une chaîne polyméthylène $-(CH_2)_3-$.

4. Composé selon la revendication 1, caractérisé par le fait que $R_1$ et $R_2$ forment ensemble un cycle aromatique, lesdits composés étant des dérivés de la benzoisothiazolone ou de l'isothiazolopyridinone.

5. Procédé de préparation des composés selon l'une des revendications 1 à 4, caractérisé par le fait que l'on fait réagir une isothiazolone de formule (X) ou (XI):

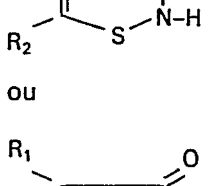

X

ou

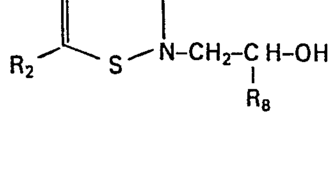

XI

sur le chlorure d'acide de l'acide rétinoïque.

6. Procédé selon la revendication 5, caractérisé par le fait que la réaction est effectuée, à la température ordinaire, en présence d'environ 1 équivalent basique, dans un solvant aromatique ou dans un éther.

7. Procédé selon la revendication 6, caractérisé par le fait que l'on utilise comme base la pyridine, la triéthylamine ou l'hydrure de sodium.

8. Procédé selon la revendication 5, caractérisé par le fait quela réaction est effectuée en portant une solution toluénique sensiblement équimoléculaire de chlorure de rétinoyle et d'izothiazolone à une température comprise entre 70 °C et 80 °C.

9. Composition médicamenteuse, caractérisée par le fait qu'elle comporte, dan sun support pharmaceutiquement acceptable, au moins un composé selon l'une des revendications 1 à 4.

10. Composition selon la revendication 9 pour le traitement des affections dermatologiques liées à un désordre de la kératinisation, des affections dermatologiques, ou autres, à composante inflammatoire et/ou immuno-allergique, des autres troubles de la kératinisation et de toutes proliférations dermatologiques bénignes ou malignes, sévères ou étendues, ou au traitement du psoriasis rhumatoïde ou au traitement d'ordre ophtalmologique.

11. Composition selon l'une des revendications 9 ou 10, administrable par voie topique, caractérisée par le fait que la concentration en composé(s) selon l'une des revendications 1 à 4 est comprise entre 0,0005% et 2% en poids.

12. Composition selon la revendication 11, caractérisée par le fait que la concentration en composé(s) selon l'une des revendications 1 à 4 est comprise entre 0,002% et 1% en poids.

13. Composition selon l'une des revendications 11 ou 12, caractérisée par le fait qu'elle est administrable sous forme d'onguent, de gel, de crème, de pommade, de poudre, de teinture, de solution, de suspension, d'émulsion, de lotion, de spray, de timbre et de tampons imbibés.

14. Composition selon l'une des revendications 9 ou 10, caractérisée par le fait qu'elle est utilisable par voie entérale.

15. Composition selon la revendication 14, caractérisée par le fait qu'elle est présentée sous forme de gélules contenant de 0,1 mg à environ 1 mg de composé(s) selon l'une des revendications 1 à 4.

16. Composition selon l'une des revendications 9 ou 10, caractérisée par le fait qu'elle se présente sous forme de solution ou suspension destinée à être administrée par voie parentérale.

17. Composition selon la revendication 16, caractérisée par le fait qu'elle contient, par ml de solution ou suspension, de 0,01 à 1 mg de composé(s) selon l'une des revendications 1 à 4.

18. Composition selon l'une des revendications 9 ou 10, caractérisée par le fait que le support pharmaceutiquement acceptable de la composition renferme au moins un produit pris dans le groupe formé par l'eau, la gélatine, le lactose, l'amidon, le talc, la vaseline, la gomme arabique, les

polyalcoylèneglycols, le stéarate de magnésium, les diluants, les solvants et les épaississants.

19. Composition cosmétique, caractérisée par le fait qu'elle contient, dans un support cosmétiquement acceptable, au moins un composé selon l'une des revendications 1 à 4.

20. Composition selon la revendication 19 pour une application en hygiène corporelle et capillaire et, notamment, dans le traitement de l'acné, des peaux physiologiquement sèches, des séborrhées, de la chute des cheveux, pour la repousse des cheveux et dans la prévention ou la protection contre les effets néfastes du soleil.

21. Composition selon l'une des revendications 19 ou 20, caractérisée par le fait que le (s) composé(s) selon l'une des revendications 1 à 4 est (ou sont) présent(s) à une concentration comprise entre 0,0005 et 2% en poids et, de préférence, entre 0,01 et 1%.

22. Composition selon l'une des revendications 19 à 21, caractérisée par le fait qu'elle se présente sous forme de lotion, de gel, de crème, de savon ou de shampooing.

23. Composition selon l'une des revendications 9 à 22, caractérisée par le fait qu'elle contient des additifs inertes ou bien pharmacodynamiquement ou cosmétiquement actifs.

24. Composition selon la revendication 23, caractérisée par le fait que les additifs sont pris dans le groupe formé par les agents hydratants, les agents anti-séborrhéiques, les agents anti-acnéiques, les antibiotiques, les agents favorisant la repousse des cheveux, les agents anti-inflammatoires, les caroténoïdes, les agents anti-psoriasiques, les agents de sapidité, les agents conservateurs, les agents stabilisants, les agents régulateurs d'humidité, les agents régulateurs de pH, les agents modificateurs de pression osmotique, les agents émulsionnants, les filtres UV-A et UV-B et les agents anti-oxydants.

## Patentansprüche

1. Chemische Verbindung der allgemeinen Formel (IV):

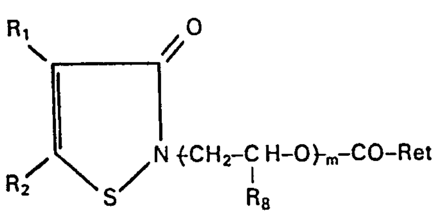

IV

worin

$R_1$ und $R_2$ gleich oder verschieden sind und

A) jeweils für ein Wasserstoffatom, einen geraden oder verzweigten $C_1$–$C_4$-Alkylrest, einen unsubstituierten Phenylrest oder einen mit einem Halogenatom, einem $C_1$–$C_4$-Alkylrest oder einem $C_1$–$C_4$-Alkoxyrest substituierten Phenylrest stehen; oder

B) zusammen genommen,

a) für eine $-(CH_2)_n$-Polymethylenkette stehen, wobei n für 3 oder 4 steht;

b) oder für eine –CH=CH–CH=CH-Kette stehen, wobei der so gebildete Benzolring mit den Resten $R_3$ und $R_4$ substituiert sein kann, die gleich oder verschieden sind und ein Wasserstoffatom, ein Halogenatom, einen $C_1$–$C_4$-Alkylrest, einen $C_1$–$C_4$-Alkoxyrest, einen Nitrorest oder einen Carboxylrest bedeuten;

c) oder für eine Verbindung der Formel (V):

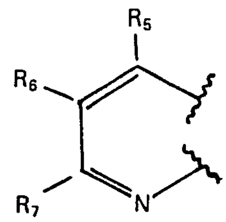

V

stehen, worin

$R_5$, $R_6$ und $R_7$ gleich oder verschieden sind und ein Wasserstoffatom oder einen geraden oder verzweigten $C_1$–$C_4$-Alkylrest bedeuten,

m für den Wert 0 oder 1 steht;

$R_8$ ein Wasserstoffatom, einen Methylrest, einen Hydroxymethylrest oder eine $-CH_2-O-CO-$Ret-Gruppe bedeutet, und

Ret für einen Rest des Retinoesäuremoleküls steht, bei dem die Säurefunktion entfernt wurde.

2. Verbindung nach Anspruch 1, dadurch gekennzeichnet, dass $R_1$ für ein Wasserstoffatom steht und $R_2$ ausgewählt ist unter einem Wasserstoffatom und einem $CH_3$- oder $C_6H_5$-Rest.

3. Verbindung nach Anspruch 1, dadurch gekennzeichnet, dass $R_1$ und $R_2$ eine $-(CH_2)_3$-Polymethylenkette bilden.

4. Verbindung nach Anspruch 1, dadurch gekennzeichnet, dass $R_1$ und $R_2$ zusammen einen aromatischen Ring bilden, wobei die Verbindungen Benzoisothiazolon- oder Isothiazolopyridinon-Derivate darstellen.

5. Verfahren zur Herstellung der Verbindungen nach einem der Ansprüche 1 bis 4, dadurch gekennzeichnet, dass man ein Isothiazolon der Formel (X) oder (XI):

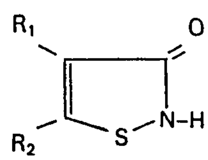

X

oder

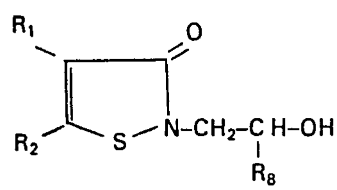

XI

mit Retinoesäurechlorid reagieren lässt.

6. Verfahren nach Anspruch 5, dadurch gekennzeichnet, dass die Reaktion bei gewöhnlicher Temperatur und in Anwesenheit von ca. einem Basenäquivalent in einem aromatischen Lösungsmittel oder in Äther durchgeführt wird.

7. Verfahren nach Anspruch 6, dadurch gekennzeichnet, dass als Base Pyridin, Triethylamin oder Natriumhydrid verwendet wird.

8. Verfahren nach Anspruch 5, dadurch gekennzeichnet dass die Reaktion durchgeführt wird, indem man eine Toluollösung von im wesentlichen äquimolaren Mengen an Retinoylchlorid und Isothiazolon auf eine Temperatur zwischen 70 °C und 80 °C bringt.

9. Arzneimittel, dadurch gekennzeichnet, dass es in einem pharmazeutisch verträglichen Träger mindestens eine Verbindung nach einem der Ansprüche 1 bis 4 umfasst.

10. Mittel nach Anspruch 9 zur Behandlung von dermatologischen Affektionen, die mit einer Störung der Keratinisierung verbunden sind, von dermatologischen Affektionen oder anderen inflammatorischen und/oder immun-allergischen dermatologischen Affektionen, von anderen Störungen der Keratinisierung sowie von allen gut- oder bösartigen, schweren oder langwierigen dermatologischen Proliferationen, oder zur Behandlung von rheumatischer Psoriasis oder zur ophthalmologischen Behandlung.

11. Mittel nach einem der Ansprüche 9 oder 10 zur topischen Verabreichung, dadurch gekennzeichnet, dass die Konzentration der Verbindung(en) nach einem der Ansprüche 1 bis 4 zwischen 0,0005 und 2 Gew.-% ausmacht.

12. Mittel nach Anspruch 11, dadurch gekennzeichnet, dass die Konzentration der Verbindung(en) nach einem der Ansprüche 1 bis 4 zwischen 0,002 und 1 Gew.-% ausmacht.

13. Mittel nach einem der Ansprüche 11 oder 12, dadurch gekennzeichnet, dass es als Salbe, Gel, Crème, Pomade, Puder, Tinktur, Lösung, Suspension, Emulsion, Lotion, Spray, als getränktes Pflaster oder als getränkter Tampon verabreichbar ist.

14. Mittel nach einem der Ansprüche 9 oder 10, dadurch gekennzeichnet, dass es enteral verwendbar ist.

15. Mittel nach Anspruch 14, dadurch gekennzeichnet, dass es in Form von Kapseln vorliegt, welche 0,1 mg bis ca. 1 mg der Verbindung(en) gemäss einem der Ansprüche 1 bis 4 enthalten.

16. Mittel nach einem der Ansprüche 9 oder 10, dadurch gekennzeichnet, dass es als Lösung oder Suspension für die parenterale Verabreichung vorliegt.

17. Mittel nach Anspruch 16, dadurch gekennzeichnet, dass es pro ml Lösung oder Suspension 0,01 bis 1 mg der Verbindung(en) gemäss einem der Ansprüche 1 bis 4 enthält.

18. Mittel nach einem der Ansprüche 9 oder 10, dadurch gekennzeichnet, dass der pharmazeutisch verträgliche Träger des Mittels mindestens ein Produkt enthält, welches ausgewählt ist unter Wasser, Gelatine, Lactose, Stärke, Talk, Vaseline, Gummi arabicum, Polyalcoylenglycolen, Magnesiumstearat, Verdünnungsmitteln, Lösungsmitteln und Verdickungsmitteln.

19. Kosmetisches Mittel, dadurch gekennzeichnet, dass es in einem kosmetisch verträglichen Träger mindestens eine Verbindung nach einem der Ansprüche 1 bis 4 enthält.

20. Mittel nach Anspruch 19 zur Verwendung bei der Hygiene von Körper und Haar und insbesondere bei der Behandlung von Akne, physiologisch trockener Haut, Seborrhoe, Haarausfall, zur Anregung des Wachstums der Haare und zur Prevention oder zum Schutz vor den negativen Wirkungen der Sonnenbestrahlung.

21. Mittel nach einem der Ansprüche 19 oder 20, dadurch gekennzeichnet, dass die Verbindung(en) gemäss einem der Ansprüche 1 bis 4 in einer Konzentration zwischen 0,0005 und 2 Gew.-%, vorzugsweise zwischen 0,01 und 1 Gew.-% vorliegt (vorliegen).

22. Mittel nach einem der Ansprüche 19 bis 21, dadurch gekennzeichnet, dass es als Lotion, Gel, Crème, Seife oder Shampoo vorliegt.

23. Mittel nach einem der Ansprüche 9 bis 22, dadurch gekennzeichnet, dass es inerte oder pharmacodynamische oder kosmetisch aktive Additive enthält.

24. Mittel nach Anspruch 23, dadurch gekennzeichnet, dass die Additive ausgewählt werden unter Hydratisierungsmitteln, Antiseborrhoe-Mitteln, Antiakne-Mitteln, Antibiotika, Mitteln, welche das Wachstum oder Haare anregen, anti-inflammatorischen Mitteln, Korotinoiden, Antipsoriasis-Mitteln, Geschmacksstoffen, Konservierungsmitteln, Stabilisierungsmitteln, die Feuchtigkeit regulierenden Mitteln, pH-regulierenden Mitteln, den osmotischen Druck modifizierenden Mitteln, Emulgatoren, UV-A und UV-B-Filtern und Antioxidationsmitteln.

**Claims**

1. Chemical compound corresponding to the general formula (IV)

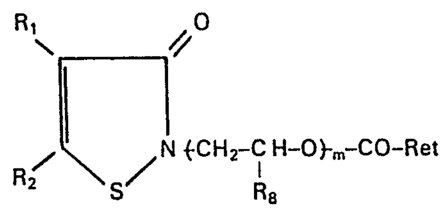

IV

in which formula $R_1$ and $R_2$ are identical or different and denote:

A) taken in isolation: a hydrogen atom, a linear or branched $C_1$–$C_4$ alkyl radical, an unsubstituted phenyl radical, or a phenyl radical substituted with a halogen atom, with a $C_1$–$C_4$ alkyl radical or with a $C_1$–$C_4$ alkoxy radical;

B) taken together:

a) either a polymethylene linkage $-(CH_2)_n-$, n being equal to 3 or 4;

b) or alternatively a $-CH=CH-CH=CH-$ linkage, the benzene ring thus formed being able to be substituted with radicals $R_3$ and $R_4$, which may be identical or different, the radicals $R_3$ and $R_4$ denoting a hydrogen atom, a halogen atom, a $C_1$–$C_4$ alkyl radical, a $C_1$–$C_4$ alkoxy radical, a nitro radical or a carboxyl radical;

c) or alternatively a linkage of formula (V)

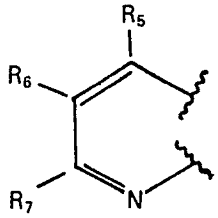

V

in which formula $R_5$, $R_6$ and $R_7$ are identical or different and denote a hydrogen atom or a linear or branched $C_1$–$C_4$ alkyl radical;

m being able to have the values 0 or 1;

$R^8$ corresponding to a hydrogen atom, a methyl radical, a hydroxymethyl radical or a $-CH_2O-CO-$Ret group;

Ret denoting the residue of the retinoic acid molecule from which the acid group has been removed.

2. Compound according to Claim 1, characterised in that $R_1$ denotes a hydrogen atom and $R_2$ is chosen from the group consisting of a hydrogen atom and a $CH_3$ or $C_6H_5$ radical.

3. Compound according to Claim 1, characterised in that $R_1$ and $R_2$ form a polymethylene chain $-(CH_2)_3-$.

4. Compound according to Claim 1, characterised in that $R_1$ and $R_2$ together form an aromatic ring, the said compounds being derivatives of benzoisothiazolone or isothiazolopyridinone.

5. Process for preparing the compounds according to one of Claims 1 to 4, characterised in that an isothiazolone for formula (X) or (XI):

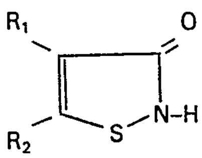

X

or

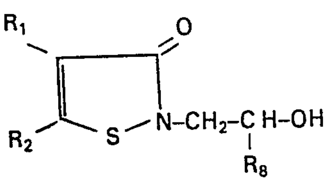

XI

is reacted with theacid chloride of retinoic acid.

6. Process according to Claim 5, characterised in that the reaction is performed at room temperature in the presence of approximately 1 equivalent of base, in an aromatic solvent or in an ether.

7. Process according to Claim 6, characterised in that pyridine, triethylamine or sodium hydride is used as base.

8. Process according to Claim 5, characterised in that the reaction is performed by bringing a substantially equimolecular toluene solution of retinoyl chloride and isothiazolone to a temperature between 70 °C and 80 °C.

9. Drug composition, characterised in that it contains, in a pharmaceutically acceptable carrier, at least one compound according to one of Claims 1 to 4.

10. Composition according to Claim 9, for treating dermatological conditions linked to a disorder of keratinisation, dermatological, or other, conditions having an inflammatory and/or immuno-allergic component, other disorders of keratinisation, and all benign or malignant, severe or extensive dermatological proliferations, or for treating rheumatoid psoriasis, or for treatment of an opthalmic nature.

11. Composition according to one of Claims 9 or 10, which can be administered topically, characterised in that the concentration of compound(s) according to one of Claims 1 to 4 is between 0.0005% and 2% by weight.

12. Composition according to Claim 11, characterised in that the concentration of compound(s) according to one of Claims 1 to 4 is between 0.002% and 1% by weight.

13. Composition according to one of Claims 11 or 12, characterised in that it can be administered in the form of ointment, gel, cream, pomade, powder, dye, solution, suspension, emulsion, lotion, spray, patch and impregnated pads.

14. Composition according to one of Claims 9 or 10, characterised in that it can be used enterally.

15. Composition according to Claim 14, characterised in that it takes the form of gelatin capsules containing from 0.1 mg to approximately 1 mg of compound(s) according to one of Claims 1 to 4.

16. Composition according to one of Claims 9 or 10, characterised in that it takes the form of solution or suspension intended to be administered parenterally.

17. Composition according to Claim 16, characterised in that it contains, per ml of solution or suspension, from 0.01 to 1 mg of compound(s) according to one of Claims 1 to 4.

18. Composition according to one of Claims 9 or 10, characterised in that the pharmaceutically at least one product chosen from the group consisting of water, gelatin, lactose, starch, talc, vaseline, gum arabic, polyalkylene glycols, magnesium stearate, diluents, solvents and thickeners.

19. Cosmetic composition, characterised in that it contains, in a cosmetically acceptable carrier, at least one compound according to one of Claims 1 to 4.

20. Composition according to Claim 19, for application in body and hair hygiene, and in particular in the treatment of acne, physiologically dry skin, seborrhoea and hair loss, to promote the regrowth of hair and in the prevention of or protection against the deleterious effects of the sun.

21. Composition according to one of Claims 19 or 20, characterised in that the compound(s) according to one of Claims 1 to 4 is/are present at a concentration between 0.0005 and 2% by weight, and preferably between 0.01 and 1%.

22. Composition according to one of Claims 19 to 21, characterised in that it takes the form of lotion, gel, cream, soap or shampoo.

23. Composition according to one of Claims 9 to 22, characterised in that it contains inert or alternatively pharmacodynamically or cosmetically active additives.

24. Composition according to Claim 23, characterised in that the additives are chosen from the group consisting of hydrating agents, anti-seborrhoeic agents, anti-acne agents, antibiotics, agents promoting the regrowth of hair, anti-inflammatory agents, carotenoids, anti-psoriatic agents, flavouring agents, preservatives, stabilisers, moisture regulating agents, pH regulating agents, osmotic pressure modifying agents, emulsifiers, UV-A and UV-B filters and antioxidants.